# EUROPEAN PATENT APPLICATION

(11) **EP 4 624 878 A1**
(43) Date of publication of application: **01.10.2025**
(21) Application number: 25165243.4
(22) Date of filing: 21.03.2025
(51) Int. Cl.: G01H 11/08, G01N 5/02, G01N 29/02, G01N 29/036, G01N 29/24, G01H 13/00, G01N 29/32

(54) **SENSING DEVICE**

(30) Priority: 28.03.2024 JP 2024053790
(71) Applicant: Nihon Dempa Kogyo Co., Ltd., Tokyo 151-8569 (JP)
(72) Inventor: KUKITA, Hiroyuki, Saitama (JP)
(74) Representative: Becker, Eberhard

(57) **Abstract**

A sensing device is provided and senses a substance to be sensed in a gas around a piezoelectric resonator based on a change in an oscillation frequency of the piezoelectric resonator. The sensing device includes: the piezoelectric resonator to which the substances to be sensed is attached; a first oscillator circuit configured to oscillate the piezoelectric resonator at a first vibration order; a second oscillator circuit configured to oscillate the piezoelectric resonator at a second vibration order greater than the first vibration order; a frequency measuring unit configured to measure respective oscillation frequencies output from the first oscillator circuit and the second oscillator circuit; a temperature changing unit configured to change a temperature of the piezoelectric resonator; and a temperature controller configured to increase a temperature of the piezoelectric resonator by the temperature changing unit based on a frequency signal output from the second oscillator circuit.

## Description

### TECHNICAL FIELD

This disclosure relates to a sensing device using a piezoelectric resonator.

### DESCRIPTION OF THE RELATED ART

There has been known a thermogravimetry using a crystal unit (Quartz Thermogravimetric analysis: QTGA). Specifically, this QTGA attaches or detaches substances contained in a gas around the crystal unit to or from the crystal unit by heating or cooling the crystal unit. The QTGA is an analysis method that uses quartz crystal microbalance (QCM) that analyzes the substances on the basis of a change in an oscillation frequency of the crystal unit caused by the above.

A device that performs such QTGA includes a crystal unit, an oscillator circuit, means for changing a temperature of the crystal unit, and means for monitoring an oscillation frequency of the crystal unit, and thus, is configured to be able to sense an attachment state of the substances in the gas to the crystal unit. For example, Japanese Patent No. 6714235 describes such a device.

### SUMMARY

### [Technical problem]

When the QTGA is performed, it is sometimes necessary to dispose a module including the crystal unit for the above-described sensing device in an environment where the analysis is performed for a relatively long period of time, and to monitor the oscillation frequency while causing the substances contained in the gas to attach to the crystal unit. At that time, there is a concern that the QTGA is no longer performable due to a stop of oscillation of the crystal unit caused by an excessive attachment amount of the above-described substances. Note that, as described in detail in the detailed description, when the QTGA is performed, it is preferred that an oscillation frequency be obtained at a high vibration order in addition to an oscillation frequency being obtained at a low vibration order, but this oscillation at the high vibration order is more prone to the oscillation stop.

From the above, there sometimes needs a countermeasure of, for example, preparing a plurality of sensing sensors including a crystal unit so as to perform the analysis with a new sensing sensor substituting for the one that has been used by then before the oscillation of the crystal unit stops, or causing the substances to attach again after being detached by heating the crystal unit at a timing set in advance after measurement start. However, increasing the number of the sensing sensors to be used as described above costs efforts and expenses in analysis. Various factors, such as a temperature and a degree of vacuum in the environment where the analysis is performed and kinds of the substances to be attached, are involved in an oscillation stop of the crystal unit, and therefore, it is difficult to accurately identify the timing at which the oscillation actually stops. Accordingly, when the crystal unit is heated as described above, a timing at which the heating is performed is set relatively much earlier than the timing at which the oscillation is expected to be stopped. Therefore, an observation of the attachment state of the substances to the crystal unit fails to continue for a sufficient period, which can lead to a failure in sufficiently increasing analysis accuracy.

The device in Japanese Patent No. 6714235 is described to use fundamental waves and third harmonics of the respective oscillation frequencies of a detection crystal unit having a configuration with which substances contained in a gas may attach and a reference crystal unit to which the substances are unable to attach when a desorption temperature and a desorption rate of the gas from the crystal unit are identified. However, this Japanese Patent No. 6714235 does not describe a method for solving the above-described problem.

A need thus exists for a sensing device which is not susceptible to the drawback mentioned above.

### [Technical solution]

According to an aspect of this disclosure, there is provided a sensing device that senses a substance to be sensed contained in a gas around a piezoelectric resonator based on a change in an oscillation frequency of the piezoelectric resonator. The sensing device includes: the piezoelectric resonator to which the substance to be sensed is attached, a first oscillator circuit, a second oscillator circuit, a frequency measuring unit, a temperature changing unit, and a temperature controller. The first oscillator circuit is configured to oscillate the piezoelectric resonator at a first vibration order. The second oscillator circuit is configured to oscillate the piezoelectric resonator at a second vibration order greater than the first vibration order. The frequency measuring unit is configured to measure respective oscillation frequencies output from the first oscillator circuit and the second oscillator circuit. The temperature changing unit is configured to change a temperature of the piezoelectric resonator. The temperature controller is configured to increase a temperature of the piezoelectric resonator by the temperature changing unit based on a frequency signal output from the second oscillator circuit.

According to another aspect of this disclosure, the temperature controller is configured to increase the temperature of the piezoelectric resonator by the temperature changing unit based on an output stop of the frequency signal from the second oscillator circuit.

According to another aspect of this disclosure, the first oscillator circuit is configured to oscillate the piezoelectric resonator with a fundamental wave, and the second oscillator circuit is configured to oscillate the piezoelectric resonator at a third harmonic.

According to another aspect of this disclosure, the piezoelectric resonator is shared between the first oscillator circuit and the second oscillator circuit, and is oscillated in a time sharing manner.

According to another aspect of this disclosure, the piezoelectric resonator includes a first vibrating region and a second vibrating region, the sensing device further comprises a cover, configured to cover the second vibrating region to block the substance to be sensed from being attached to the second vibrating region, and the first vibrating region and the second vibrating region are oscillated in a time sharing manner.

### [Technical effect]

With a sensing device according to an aspect of this disclosure, the temperature of the piezoelectric resonator is increased so as to avoid the obtainment of the frequency signal from the first oscillator circuit that oscillates the piezoelectric resonator at a low vibration order from being disabled, and the timing at which this temperature increase is allowed to be prevented from being performed much earlier than the timing at which the obtainment of the frequency signal from this first oscillator circuit is disabled. Therefore, the transition of the frequency signal from the first oscillator circuit before the above-described temperature increase is performed is observable over a long period of time, the substances to be sensed are allowed to be sensed with high sensitivity using the frequency signal from the second oscillator circuit, and an operation abortion caused by disablement of obtaining both the frequency signals from the first and second oscillator circuits is avoidable. The sensing device according to the aspect of this disclosure is highly convenient because of such advantages.

### BRIEF DESCRIPTION OF THE DRAWINGS

The foregoing and additional features and characteristics of this disclosure will become more apparent from the following detailed description considered with reference to the accompanying drawings, wherein:
FIG. 1 is a longitudinal sectional side view of a sensing device according to one embodiment disclosed here;
FIG. 2 is a block diagram of the sensing device;
FIG. 3 is a block diagram illustrating a connection between a crystal unit and an oscillator circuit in the sensing device; and
FIG. 4 is a graph diagram for describing an exemplary operation of the sensing device.

### DETAILED DESCRIPTION

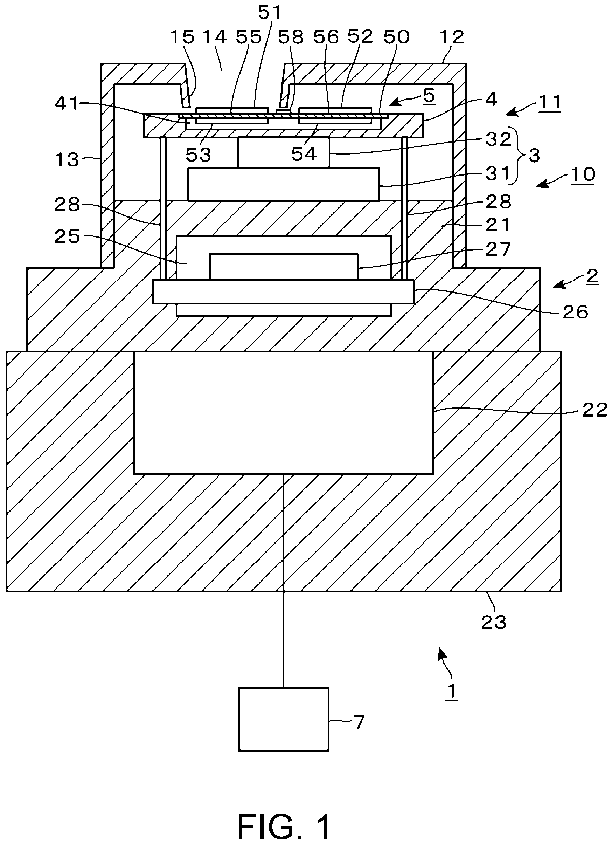

A description will be given of a sensing device 1 as one embodiment of the present disclosure with reference to a longitudinal sectional side view in FIG. 1. The sensing device 1 is a device for performing QTGA, and is configured of a sensing sensor 10 including a crystal unit 5 and oscillator circuits, a connection member 23 to/from which the sensing sensor 10 is attached and removed, and a control unit 7 that detects an oscillation frequency and supplies an electric power to each unit of the sensing sensor 10.

The sensing sensor 10 is configured of a base 2, a Peltier element unit 3, and a crystal unit holder 4 arranged in this order, and the members adjacent to one another among these members are connected to one another. A description will be given below with the base 2 and the crystal unit holder 4 being positioned in a lower side and an upper side, respectively upon describing a configuration of the sensing sensor 10. However, the directions in this description do not limit the location of the sensing sensor 10, and any direction is allowed for the sensing sensor 10 in use. The above-described crystal unit 5 is supported from the lower side by the crystal unit holder 4.

The base 2 is configured as a circular block in plan view. A center portion projects upward to form a mounting block 21 in a circular shape, and the Peltier element unit 3 is disposed on the mounting block 21. The base 2 has a lower surface that also projects to form a square connector 22. The connector 22 is inserted into a depressed portion 24 of the connection member 23, and thus, a terminal formed on the connector 22 is electrically connected to the control unit 7 via a conductive path (not illustrated) formed in the connection member 23. The connection member 23 is cooled by a cooling mechanism (not illustrated), and cools this base 2 by being brought into contact with the base 2 from the lower side with the connector 22 being inserted into the depressed portion 24.

The above-described Peltier element unit 3 is a temperature changing unit that heats and cools the crystal unit 5 as a piezoelectric resonator via the crystal unit holder 4, and is configured of a first Peltier element 31 and a second Peltier element 32 stacked together. The first Peltier element 31 is disposed in a lower side and the second Peltier element 32 is disposed in an upper side. The first Peltier element 31 and the second Peltier element 32 have upper surfaces and lower surfaces, where the upper surfaces serve as an endothermic surface and the lower surfaces serve as a heat dissipation surface when the crystal unit 5 is cooled, and the upper surfaces serve as the heat dissipation surface and the lower surfaces serve as the endothermic surface when the crystal unit 5 is heated. Thus, since the heat dissipation surface of the second Peltier element 32 is cooled by the first Peltier element 31 when the crystal unit 5 is cooled, the Peltier element unit 3 is able to cool the crystal unit 5 down to a relatively low temperature, and the heat generated from the heat dissipation surface of the first Peltier element 31 during the cooling is dissipated to the connection member 23 via the base 2. This Peltier element unit 3 changes the temperature of the crystal unit 5 within a range of, for example, -80°C to +125°C.

The center portion in plan view of the base 2 has a sealed space 25, and a circuit board 26 is disposed in the sealed space 25. Note that a heat insulating member (not illustrated) is interposed between the circuit board 26 and a wall surface forming the sealed space 25 such that the circuit board 26 is not affected by the heat dissipation by the Peltier element unit 3 described above. On the circuit board 26, an integrated circuit chip (IC chip) 27 is disposed. The integrated circuit chip 27 includes a first oscillator circuit 61, a second oscillator circuit 62, and switches 63 to 65 described later.

The crystal unit holder 4 is configured as a substrate in a horizontal posture including a depressed portion 41 that opens upward. This depressed portion 41 has an opening edge that supports a peripheral edge portion of the crystal unit 5 from the lower surface side and holds this crystal unit 5 in a horizontal posture such that lower surfaces of a first vibrating region 55 and a second vibrating region 56 of the crystal unit 5 described later face the depressed portion 41.

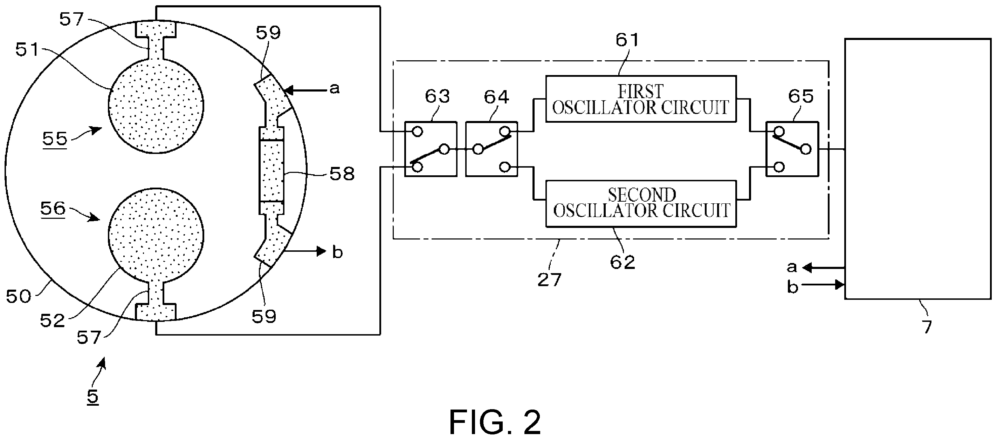

Subsequently, with reference to FIG. 2 as well, a configuration of the crystal unit 5 will be described. FIG. 2 illustrates an upper surface of the crystal unit 5 and is also a block diagram illustrating a configuration of an integrated circuit chip 27. The crystal unit 5 includes, for example, a crystal element 50 in a circular shape as an AT-cut piezoelectric piece. On one side of a surface (upper surface side) and another side of the surface (lower surface side) of this crystal element 50, a pair of first excitation electrodes (detection electrodes) 51, 53 and a pair of second excitation electrodes (reference electrodes) 52, 54 configured of, for example, gold (Au) are disposed to be spaced apart from one another. In the crystal element 50, a region between the first excitation electrodes 51, 53 configures a first vibrating region 55 and a region between the second excitation electrodes 52, 54 configures a second vibrating region 56, and these first vibrating region 55 and second vibrating region 56 are able to individually vibrate.

The excitation electrodes 51 to 54 are in circular shapes, and have parts of edges of the circles extracted toward a peripheral edge of the crystal unit 5, thus configuring extraction electrodes 57. For example, a resistor of platinum (Pt) is disposed as a temperature sensor 58 on the crystal element 50, and both ends of the temperature sensor 58 are connected to conductive patterns 59 made of Au formed on the crystal element 50. For the temperature sensor 58, the control unit 7 supplies a current and detects a resistance value, and the control unit 7 detects a temperature of the crystal unit 5 based on the detected resistance value. The extraction electrode 57 and the conductive pattern 59 are connected to terminals of the circuit board 26 via a conductive pattern disposed on the crystal unit holder 4 and rod-shaped conductive members 28 vertically extending between the crystal unit holder 4 and the circuit board 26.

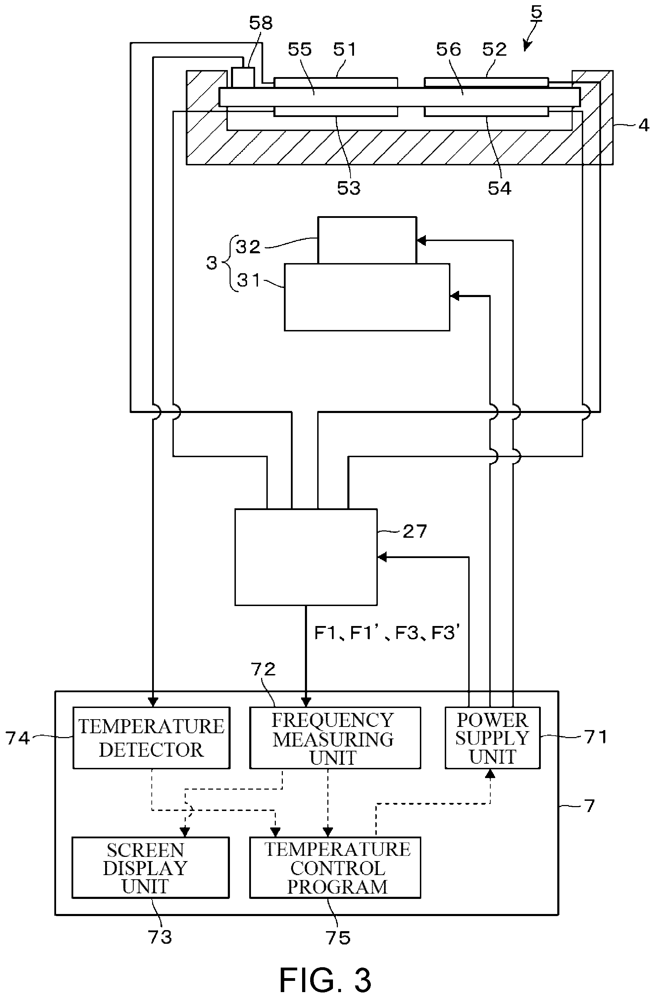

Note that the terminal of the connector 22 of the base described above is electrically connected to the respective terminals of the Peltier element unit 3 and the circuit board 26 via a conductive path disposed in the base 2. As described above, the connector 22 is inserted into the depressed portion 24 of the connection member 23 and the connection between the terminal of this connector 22 and the control unit 7 is established, and thus, the integrated circuit chip 27 on the circuit board 26, the temperature sensor 58, the excitation electrodes 51 to 54 configuring the crystal unit 5, and the Peltier element unit 3 are electrically connected to the control unit 7. FIG. 3 is a block diagram illustrating a thus connected state.

The sensing sensor 10 includes a cover 11. The cover 11 includes a main portion 12 in a circular shape in plan view that covers an upper side of the crystal unit 5 and the crystal unit holder 4, and a cylindrical portion 13 that extends downward from a peripheral edge of the main portion 12 and surrounds a lateral side of the crystal unit holder 4 and the Peltier element unit 3, and the cylindrical portion 13 has a lower end brought into contact with an outer side of the mounting block 21 on the base 2. The main portion 12 has a circular through hole 14 formed at a position overlapping with the first excitation electrode 51 in plan view, the through hole 14 has a peripheral edge portion extended downward to form a cylindrical-shaped shield 15 brought close to the first excitation electrode 51. Thus forming the cover 11 causes substances to be sensed contained in a gas around the sensing sensor 10 to attach to the excitation electrode 51 among the excitation electrodes 51 to 54 (for the vibrating region, the first vibrating region 55 among the first vibrating region 55 and the second vibrating region 56) in a limited way.

The sensing device 1 obtains each of an oscillation frequency of the first vibrating region 55 to which the substances to be sensed contained in the gas attach as described above and an oscillation frequency of the second vibrating region 56 to which the substances to be sensed do not attach using the control unit 7. Calculating a difference between these oscillation frequencies respectively output from the first vibrating region 55 and the second vibrating region 56 allows cancelling an influence of a temperature change around the sensing sensor 10 and highly accurately detecting an attachment state of the substances to be sensed to the first vibrating region 55. Accordingly, the first vibrating region 55 is a vibrating region for detecting the substances to be sensed and the second vibrating region is a vibrating region for reference.

To describe the obtainment of the oscillation frequencies in more detail, this sensing device 1 switches an oscillation at fundamental waves and an oscillation at third harmonics (third overtone of the fundamental waves) in a time sharing manner for each of the first vibrating region 55 and the second vibrating region 56. Accordingly, the device configuration enables obtaining each of the oscillation frequency of the fundamental waves in the first vibrating region 55 and the oscillation frequency of the fundamental waves in the second vibrating region 56 and calculating a difference between them, and enables obtaining each of the oscillation frequency of the third harmonics in the first vibrating region 55 and the oscillation frequency of the third harmonics in the second vibrating region 56 and calculating a difference between them.

The first oscillator circuit 61 included in the integrated circuit chip 27 described above oscillates the first vibrating region 55 and the second vibrating region 56 at the fundamental waves, and the second oscillator circuit 62 oscillates the first vibrating region 55 and the second vibrating region 56 at the third harmonics. Specifically, for example, at ordinary temperature, the first oscillator circuit 61 oscillates the first vibrating region 55 and the second vibrating region 56 at 10 MHz and the second oscillator circuit 62 oscillates the first vibrating region 55 and the second vibrating region 56 at 30 MHz.

The reasons of thus performing the oscillations at the fundamental waves having a vibration order of 1 and at the third harmonics having a vibration order of 3 are as follows. The larger the vibration order is, the larger the change amount of the frequency with respect to an attachment amount of the substances to be sensed to the excitation electrode becomes, and therefore, detection sensitivity of the substances to be sensed can be enhanced. However, the larger the vibration order is, the lower the negative resistance of the oscillator circuit becomes, and therefore, an oscillation margin is reduced. This means the range of the frequency in which the measurement can be taken is small because the amount of the substances to be sensed attachable to the excitation electrode is reduced before the oscillation stops.

That is, depending on the amount of the gas in the environment where the sensing sensor 10 is disposed, it differs which one of the fundamental waves and the third harmonics is advantageously used for performing the QTGA. To give a specific example, in order to observe a discharge condition of a gas (outgas) emitted from members (components, adhesive agents, and the like) configuring a system used in the outer space, such as an artificial satellite, the members and the sensing sensor 10 are installed in an applicable environment and frequency measurements are taken. If a trace of the outgas is discharged from this member, upon observing the discharge condition of the outgas, using the oscillation frequency at the third harmonics is advantageous because the discharge condition can be highly accurately obtained. On the other hand, when the discharge amount of the outgas is relatively large, observation of the discharge condition of the outgas using the oscillation frequency at the fundamental waves with which a stop of oscillation output is less likely to occur even though a large amount of substances attach to the excitation electrode is advantageous.

Therefore, the sensing device 1 is configured to obtain respective oscillation frequencies at the fundamental waves and the third harmonics as described above. As the deposition of the substances contained in the gas on the first excitation electrode 51 proceeds, for the first vibrating region 55, this sensing device 1 heats the crystal unit 5 and increases the temperature of the crystal unit 5 on the basis of the timing of the oscillation stop at the third harmonics by using a property of the oscillation at the third harmonics that it stops prior to the oscillation at the fundamental waves. Thus, it is configured that the oscillation stop at the fundamental waves is avoided. To describe even more specifically, the control unit 7 configuring the sensing device 1 detects presence/absence of an output of a frequency signal of the third harmonics in the first vibrating region 55 to the control unit 7. That is, the detection of whether the vibration at the third harmonics in the first vibrating region 55 is stopped or not is performed. When the output stop of the frequency signal at the third harmonics is detected, the configuration causes the control unit 7 to increase the temperature of the crystal unit 5 by the Peltier element unit 3 to detach the substances to be sensed attached to the first excitation electrode 51, and avoid the oscillation stop at the fundamental waves in the first vibrating region 55, such that the obtainment of the oscillation frequency at the fundamental waves is continuable.

Return to FIG. 2, each element disposed in the integrated circuit chip 27 will be described. The switch 63 is disposed downstream of the first excitation electrode 51, 53, and the second excitation electrodes 52, 54, and the switch 64 is disposed in a position after the switch 63. Note that FIG. 2 omits illustrations of the first excitation electrode 53 and the second excitation electrode 54 on the lower surface side. The first oscillator circuit 61 and the second oscillator circuit 62 are disposed in positions after the switch 64, the switch 65 is disposed in a position after the first oscillator circuit 61 and the second oscillator circuit 62, and the control unit 7 is disposed in a position after the switch 65.

The switches 63, 64 switch a state where the first vibrating region 55 oscillates at the fundamental waves with the first excitation electrodes 51, 53 being connected to the first oscillator circuit 61, a state where the first vibrating region 55 oscillates at the third harmonics with the first excitation electrodes 51, 53 being connected to the second oscillator circuit 62, a state where the second vibrating region 56 oscillates at the fundamental waves with the second excitation electrodes 52, 54 being connected to the first oscillator circuit 61, and a state where the second vibrating region 56 oscillates at the third harmonics with the second excitation electrodes 52, 54 being connected to the second oscillator circuit 62 in sequence in a time sharing manner, and this switching is repeatedly performed. The switch 65 operates by synchronizing with the operations of the switches 63, 64, and the connection of the first oscillator circuit 61 to the control unit 7 and the connection of the second oscillator circuit 62 to the control unit 7 are alternately switched in a time sharing manner.

With the operations of the switches 63 to 65 described above, the first vibrating region 55 and the second vibrating region 56 oscillate in a time sharing manner, and a frequency signal of the fundamental waves from the first vibrating region 55, a frequency signal of the third harmonics from the first vibrating region 55, a frequency signal of the fundamental waves from the second vibrating region 56, and a frequency signal of the third harmonics from the second vibrating region 56 are repeatedly output to the control unit 7 in an order in a time sharing manner. Subsequently, for the frequencies of the signals output from the first oscillator circuit 61, the frequency at the fundamental waves in the first vibrating region 55 is described as a fundamental wave detection frequency F1, and the frequency at the fundamental waves in the second vibrating region 56 is described as a fundamental wave reference frequency F1'. For the frequencies of the signals output from the second oscillator circuit 62, the frequency at the third harmonics in the first vibrating region 55 is described as a third harmonic detection frequency F3, and the frequency at the third harmonics in the second vibrating region 56 is described as a third harmonic reference frequency F3'.

Subsequently, the control unit 7 that configures a temperature controller will be described with reference to FIG. 3. The control unit 7 includes a power supply unit 71, a frequency measuring unit 72, a screen display unit 73, a temperature detector 74, and a temperature control program 75. While the power supply unit 71 supplies an electric power to each units of the device, such as the integrated circuit chip 27, the Peltier element unit 3, and the temperature sensor 58, FIG. 3 illustrates as if only the integrated circuit chip 27 and the Peltier element unit 3 are supplied with the electric power for convenience. The frequency measuring unit 72 receives a frequency signal output from the integrated circuit chip 27, and measures each of the fundamental wave detection frequency F1, the fundamental wave reference frequency F1', the third harmonic detection frequency F3, and the third harmonic reference frequency F3'. The screen display unit 73 displays the fundamental wave detection frequency F1, the fundamental wave reference frequency F1', the third harmonic detection frequency F3, and the third harmonic reference frequency F3' measured by the frequency measuring unit 72 on the screen.

The temperature detector 74 detects a temperature of the crystal unit 5 from a resistance value of the temperature sensor 58 as described above. The temperature control program 75 monitors the third harmonic detection frequency F3 and controls the electric power supplied to the Peltier element unit 3 from the power supply unit 71, and thus, controls the temperature of the crystal unit 5. This temperature control program 75 performs a temperature control of the crystal unit 5 in the case where the obtainment of the above-described third harmonic detection frequency F3 fails. When the temperature control of the crystal unit 5 is thus performed, the detected value by the temperature detector 74 is used. The exemplary operation of the device described later will describe an exemplary temperature control of the crystal unit executed by this temperature control program 75.

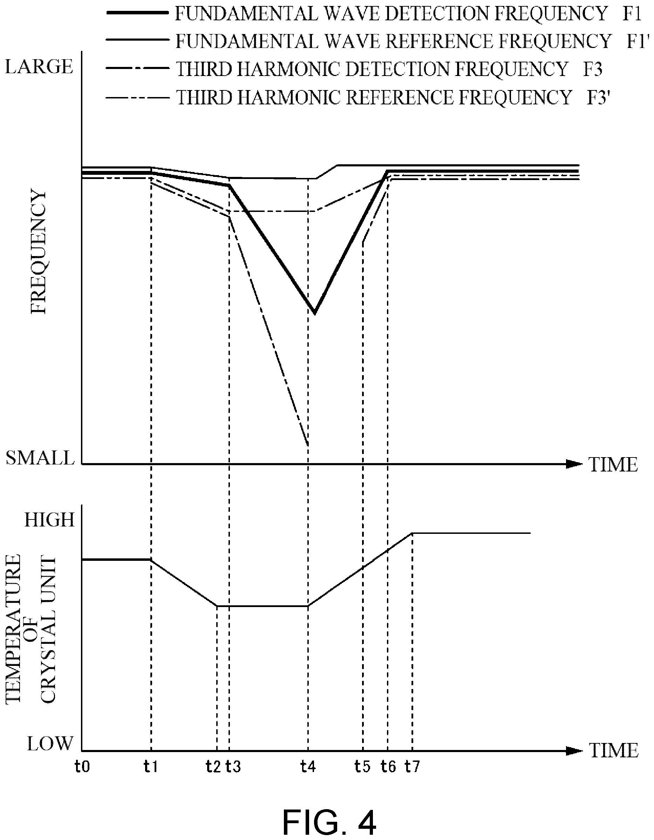

Subsequently, with reference to FIG. 4, one example of the operation of the sensing device 1 will be described. FIG. 4 is a chart diagram illustrating transitions of the fundamental wave detection frequency F1, the fundamental wave reference frequency F1', the third harmonic detection frequency F3, and the third harmonic reference frequency F3' obtained during the measurements correlated with a transition of the temperature of the crystal unit 5 obtained by the temperature sensor 58 during the measurements.

In this measurement, the sensing sensor 10 and a member as a measurement target are installed in an evacuated measurement environment, and a discharge condition of the outgas from the member as the measurement target is observed. There is provided, for example, an openable/closable shutter in the measurement environment, which enables switching between a state of separating the member as the measurement target and the sensing sensor 10 by closing the shutter and a state of not separating the member as the measurement target and the sensing sensor 10 by opening the shutter.

At Time t0 in the chart, obtainment of the fundamental wave detection frequency F1, the fundamental wave reference frequency F1', the third harmonic detection frequency F3, and the third harmonic reference frequency F3' by the control unit 7 is started. At subsequent Time t1, cooling of the crystal unit 5 by the Peltier element unit 3 is started, and the temperature of the crystal unit 5 drops. Oscillation characteristics of the crystal unit 5 are changed by the temperature change, and each of the frequencies F1, F1', F3, F3' is changed.

The temperature of the crystal unit 5 is further decreased as the cooling by the Peltier element unit 3 is continued, and when the crystal unit 5 is cooled down to a temperature set in advance (Time t2), this temperature is maintained. The shutter of the measurement space is opened, the outgas discharged from the member as the measurement target and supplied to a peripheral area of the crystal unit 5 is cooled, and the substances to be sensed contained in this gas attach to the first excitation electrode 51. That attachment decreases the fundamental wave detection frequency F1 and the third harmonic detection frequency F3 (Time t3). Note that the third harmonic detection frequency F3 has a larger reduction amount per unit time than the fundamental wave detection frequency F1.

The attachment of the substances to be sensed contained in the gas proceeds, which causes the decrease of the fundamental wave detection frequency F1 and the third harmonic detection frequency F3 to proceed. The excessive attachment stops the oscillation with the third harmonics in the first vibrating region 55, and therefore, the obtainment of the third harmonic detection frequency F3 is disabled (Time t4). Note that, as described above, the oscillation with high-order harmonics stops prior to the oscillation with low-order harmonics, and therefore, the obtainment of the fundamental wave detection frequency F1 is continuing at this oscillation stop at the third harmonics.

The third harmonic detection frequency F3 is no longer obtained, which causes the cooling of the crystal unit 5 by the Peltier element unit 3 to stop, and thus, the heating of the crystal unit 5 is started (Time t4). The temperature increase of the crystal unit 5 detaches and removes the substances to be sensed attached to the first excitation electrode 51 into the measurement space, and the fundamental wave detection frequency F1 increases. The desorption of the substances to be sensed thus proceeding enables the first vibrating region 55 to oscillate with the third harmonics again, and the third harmonic detection frequency F3 is obtained again (Time t5). Since the desorption of the substances to be sensed is continuing, the third harmonic detection frequency F3 also increases similarly to the fundamental wave detection frequency F1. When the desorption of the substances to be sensed is completed, the fundamental wave detection frequency F1 and the third harmonic detection frequency F3 are brought into a state of transitioning in a constant or mostly constant manner (Time t6). When the temperature of the crystal unit 5 reaches the temperature set in advance, the temperature increase of the crystal unit 5 stops (Time t7). The temperature control of the crystal unit 5 from Time t4 to Time t7 is executed by the above-described temperature control program 75.

For example, at and after Time t4 at which the temperature of the crystal unit 5 is increased, the sensing sensor 10 and the measurement target are separated by closing the shutter of the measurement space, each of the frequencies F1, F1', F3, F3' is obtained again by performing the operations at and after Time t0 again, and the frequencies F1, F1', F3, F3' may be used for the analysis together with each frequency that has been obtained by then, or only each of the frequencies F1, F1', F3, F3' that has been obtained by Time t4 may be used for the analysis. Note that, in performing the analysis, in order to cancel the influence of the temperature change around the crystal unit 5 to the frequency as described above, F1 - F1', F3 - F3' at each time are calculated, and time-series data of these F1 - F1' and time-series data of these F3 - F3' may be used. The control unit 7 may be configured to calculate each of the time-series data of these F1 - F1', F3 - F3', and be able to display them.

As described above, with the sensing device 1, heating the crystal unit 5 and increasing the temperature of the crystal unit 5 on the basis of the presence/absence of the obtainment of the third harmonic detection frequency F3 (that is, the presence/absence of the oscillation of the first vibrating region 55 at the third harmonics) enables avoiding the output stop of the fundamental wave detection frequency F1 and enables avoiding the timing of this heating from becoming excessively early with respect to the timing of the output stop of the fundamental wave detection frequency F1. Therefore, it is highly convenient as a device.

While in the example shown in the chart in FIG. 4, the heating of the crystal unit 5 is started by increasing the temperature by the Peltier element unit 3 at the same time as the disablement of obtaining the third harmonic detection frequency F3, the timing of the heating of the crystal unit 5 is not limited to be performed at the same time as the disablement of obtaining the third harmonic detection frequency F3. For example, the heating may be started after a lapse of time set in advance from the timing where the obtainment of the third harmonic detection frequency F3 is disabled.

The reduction amount of the third harmonic detection frequency F3 per unit time is larger than the reduction amount of the fundamental wave detection frequency F1 per unit time. Therefore, the heating of the crystal unit 5 may be started when the third harmonic detection frequency F3 decreases after the measurement is started, and the frequency set in advance (a reference frequency) is reached. The heating of the crystal unit 5 may be performed when the third harmonic detection frequency F3 has decreased by a predetermined amount. Accordingly, controlling the timing at which the heating of the crystal unit 5 is performed on the basis of the third harmonic detection frequency F3 is not limited to be triggered by the disablement of obtaining the third harmonic detection frequency F3 (the oscillation at the third harmonics stops) as the example that has been described so far. However, heating the crystal unit 5 in a state where the oscillation at the third harmonics is being performed as such possibly perform the heating relatively much earlier than the timing at which the obtainment of the fundamental wave detection frequency F1 is disabled. Therefore, from the aspect of extending the time period of obtaining the fundamental wave detection frequency F1 before heating the crystal unit 5, it is preferred that the heating of the crystal unit 5 be performed triggered by the disablement of obtaining the third harmonic detection frequency F3 as described so far.

The example of obtaining the frequencies at the fundamental waves and the third harmonics has been described, the obtained frequencies are not limited to the frequencies at the fundamental waves or the third harmonics. For example, the device configuration may obtain the fundamental waves and fifth harmonics (the vibration order is 5) or may obtain the third harmonics and the fifth harmonics. As described above, in deposition of the substances to be sensed on the excitation electrode of the crystal unit, an oscillation with high-order harmonics stops prior to an oscillation with low-order harmonics. Therefore, when the fundamental waves and the fifth harmonics are used, the heating of the crystal unit 5 may be performed triggered by the oscillation stop at the fifth harmonics having the large vibration order, and when the third harmonics and the fifth harmonics are used, the heating of the crystal unit 5 may also be performed triggered by the oscillation stop at the fifth harmonics having the large vibration order. However, the larger the vibration order is, the smaller the amount of the substances to be sensed that can attach while vibrating the first vibrating region 55 as described so far becomes. Accordingly, from the aspect of observing the state of the substances to be sensed around the sensing sensor by performing the frequency measurement for a relatively long period of time, it is preferred that the fundamental waves and the third harmonics be used.

The sensing device is not limited to have configuration of including the Peltier element unit 3. To give a specific example, a rod-shaped supporting member extending upward is disposed on the base 2 instead of the Peltier element unit 3, this supporting member supports the crystal unit holder 4, and a temperature-changeable heater is disposed in the crystal unit holder 4. The connection member 23 into which the connector 22 of the base 2 is inserted may have a configuration of being cooled to a constant relatively low temperature by, for example, liquid nitrogen, which enables also the crystal unit 5 to be cooled to a relatively low temperature by heat transfer via the base 2, the supporting member, and the crystal unit holder 4. When the device is thus configured, the heater of the crystal unit holder 4 is the temperature changing unit, and the temperature of this heater being changed changes the temperature of the crystal unit 5.

In the sensing device 1 described above, the crystal unit that vibrates with the fundamental waves and the crystal unit that vibrates with the third harmonics are the same as one another. That is, the configuration has the crystal unit 5 being shared between the first oscillator circuit 61 and the second oscillator circuit 62, however, the configuration may have the crystal unit disposed for each oscillator circuit. When the temperature of the crystal unit 5 is increased, the supplied electric power to the Peltier element unit 3 may be reduced to lower the cooling performance of this Peltier element unit 3. That is, the configuration is not limited to increasing the temperature by heating the crystal unit 5.

The embodiment disclosed this time is illustrative in every point and should be considered not to be restrictive. The above-described embodiment may be omitted, replaced, changed, and combined in various manners without departing from accompanying claims and their spirits.

The principles, preferred embodiment and mode of operation of the present invention have been described in the foregoing specification. However, the invention which is intended to be protected is not to be construed as limited to the particular embodiments disclosed. Further, the embodiments described herein are to be regarded as illustrative rather than restrictive. Variations and changes may be made by others, and equivalents employed, without departing from the spirit of the present invention. Accordingly, it is expressly intended that all such variations, changes and equivalents which fall within the spirit and scope of the present invention as defined in the claims, be embraced thereby.

### Description of Reference Numerals

1: sensing device
2: base
3: Peltier element unit
4: crystal unit holder
5: crystal unit
7: control unit
10: sensing sensor
11: cover
12: main portion
13: cylindrical portion
14: through hole
15: cylindrical-shaped shield
21: mounting block
22: connector
23: connection member
25: sealed space
26: circuit board
27: integrated circuit chip
28: rod-shaped conductive member
31: first Peltier element
32: second Peltier element
41: depressed portion
50: crystal element
51, 53: first excitation electrode
52, 54: second excitation electrode
55: first vibrating region
56: second vibrating region
57: extraction electrode
58: temperature sensor
59: conductive pattern
61: first oscillator circuit
62: second oscillator circuit
63, 64, 65: switch
71: power supply unit
72: frequency measuring unit
73: screen display unit
74: temperature detector
75: temperature control program
F1: fundamental wave detection frequency
F1': fundamental wave reference frequency
F3: third harmonic detection frequency
F3': third harmonic reference frequency
t0~t7: time

## Claims

1. A sensing device (1) that senses a substance to be sensed contained in a gas around a piezoelectric resonator (5) based on a change in an oscillation frequency of the piezoelectric resonator (5), the sensing device comprising:
the piezoelectric resonator (5) to which the substance to be sensed is attached;
a first oscillator circuit (61) configured to oscillate the piezoelectric resonator (5) at a first vibration order;
a second oscillator circuit (62) configured to oscillate the piezoelectric resonator (5) at a second vibration order greater than the first vibration order;
a frequency measuring unit (72) configured to measure respective oscillation frequencies output from the first oscillator circuit (61) and the second oscillator circuit (62);
a temperature changing unit (3) configured to change a temperature of the piezoelectric resonator (5); and
a temperature controller (7) configured to increase a temperature of the piezoelectric resonator (5) by the temperature changing unit (3) based on a frequency signal output from the second oscillator circuit (62).

2. The sensing device (1) according to claim 1, wherein
the temperature controller (7) is configured to increase the temperature of the piezoelectric resonator (5) by the temperature changing unit (3) based on an output stop of the frequency signal from the second oscillator circuit (62).

3. The sensing device (1) according to claim 2, wherein
the first oscillator circuit (61) is configured to oscillate the piezoelectric resonator (5) with a fundamental wave, and the second oscillator circuit (62) is configured to oscillate the piezoelectric resonator (5) at a third harmonic.

4. The sensing device (1) according to claim 3, wherein
the piezoelectric resonator (5) is shared between the first oscillator circuit (61) and the second oscillator circuit (62), and is oscillated in a time sharing manner.

5. The sensing device (1) according to claim 4, wherein
the piezoelectric resonator (5) includes a first vibrating region (55) and a second vibrating region (56),
the sensing device (1) further comprises a cover (11), configured to cover the second vibrating region (56) to block the substance to be sensed from being attached to the second vibrating region (56), and
the first vibrating region (55) and the second vibrating region (56) are oscillated in a time sharing manner.
